Europäisches Patentamt

European Patent Office

Office européen des brevets

(19)

(11) Veröffentlichungsnummer : **0 629 294 B1**

(12)                        **EUROPÄISCHE PATENTSCHRIFT**

(45) Veröffentlichungstag der Patentschrift :
08.11.95 Patentblatt 95/45

(21) Anmeldenummer : 93903710.7

(22) Anmeldetag : 25.02.93

(86) Internationale Anmeldenummer :
PCT/AT93/00032

(87) Internationale Veröffentlichungsnummer :
WO 93/17343 02.09.93 Gazette 93/21

(51) Int. Cl.⁶ : **G01N 33/574, G01N 33/68**

(54) **VERFAHREN ZUR IMMUNOLOGISCHEN ERKENNUNG MALIGNER TUMORE UND KIT ZUR DURCHFÜHRUNG DIESES VERFAHRENS.**

(30) Priorität : 27.02.92 AT 368/92

(43) Veröffentlichungstag der Anmeldung :
21.12.94 Patentblatt 94/51

(45) Bekanntmachung des Hinweises auf die
Patenterteilung :
08.11.95 Patentblatt 95/45

(84) Benannte Vertragsstaaten :
AT BE CH DE DK ES FR GB IE IT LI NL SE

(56) Entgegenhaltungen :
US-A- 4 219 539
DATABASE WPI, Section Ch, Week 7239, Derwent Publications Ltd., London (GB); Class
A03, AN 72-62480T/

(73) Patentinhaber : SCHAUENSTEIN, Erwin
Am Eisernen Tor 2
A-8010 Graz (AT)

Patentinhaber : SCHAUENSTEIN, Konrad
Carnerigasse 20
A-8010 Graz (AT)
Patentinhaber : DACHS, Franz
Spattendordf 10
A-4210 Gallneukirchen (AT)

(72) Erfinder : SCHAUENSTEIN, Erwin
Am Eisernen Tor 2
A-8010 Graz (AT)
Erfinder : SCHAUENSTEIN, Konrad
Carnerigasse 20
A-8010 Graz (AT)
Erfinder : DACHS, Franz
Spattendordf 10
A-4210 Gallneukirchen (AT)

(74) Vertreter : Atzwanger, Richard Dipl.Ing.
Patentanwälte Dipl.-Ing. Anton Atzwanger
Dipl.-Ing. Richard Atzwanger
Mariahilfer Strasse 1c
A-1060 Wien (AT)

EP 0 629 294 B1

## Beschreibung

Gegenstand der vorliegenden Erfindung ist ein Verfahren zur Feststellung maligner Tumore bzw. zur Bestimmung ihrer Tumormasse anhand von Eigenschaften des Serum-Immunglobulins G (IgG) des Patienten.

Seit mehreren Jahren ist es bekannt, daß bei Patienten mit malignen Tumoren eine signifikante Veränderung der Mengen der Sulfhydryl- bzw. Disulfidgruppen (gemessen mittels der Dithionitrobenzoat-Reaktion) innerhalb des Serum-IgG zu beobachten ist. Der experimentelle Meßwert für das Verhältnis von Sulfhydryl- zu Disulfidgruppen wird als ΣS-Wert bezeichnet, wobei bis vor kurzem die Ursache für die Veränderung des ΣS-Wertes unbekannt war.

Im Laufe der weiteren Untersuchungen auf diesem Gebiet zeigte sich nun, daß die Veränderung des ΣS-Wertes auf eine Verschiebung des Verhältnisses der Subklasse IgG1 zu den anderen Subklassen (z.B. IgG2) zurückzuführen ist.

Dieser Zusammenhang ist neu und gibt dem Fachmann neben der grundlegenden wissenschaftlichen Erkenntnis eine Möglichkeit der diagnostischen Nutzung dieses Phänomens.

Unabhängig davon hat sich im Zuge der diesbezüglichen Forschungsarbeiten immer wieder gezeigt, daß sich die Meßwerte bei frischen Blut- bzw. Serumproben innerhalb kurzer Zeit relativ stark verändern. Eine Durchführung der verschiedenen Analyseverfahren für IgG an unbehandelten Blut- oder Serumproben führt somit zu keinen reproduzierbaren und auswertbaren Ergebnissen.

Ziel der vorliegenden Erfindung ist es daher, während eines vernünftigen Zeitraums von mindestens einem Tag reproduzierbare Angaben über ein eventuelles Vorhandensein sowie über Ausmaß und Entwicklung eines malignen Tumors in einem Patienten zu erhalten.

Dies wird erfindungsgemäß dadurch erreicht, daß die frischen Blut- oder Serumproben des Patienten mit Radikalfängern, vorzugsweise mit Superoxiddismutase (SOD), stabilisiert werden und im Serum der Proben die Verschiebung des Verhältnisses mindestens einer der IgG-Subklassen zu der Summe der IgG-Subklassen im Vergleich zum Normalverhältnis bestimmt wird.

Vorzugsweise wird im Serum der stabilisierten Proben die Verschiebung des Verhältnisses der Subklasse IgG1 und bzw. oder der Subklasse IgG2 zu der Summe der IgG-Subklassen im Vergleich zum Normalverhältnis bestimmt.

Die hinsichtlich der Stabilität der IgG1-Werte durchgeführten Untersuchungen unbehandelter Proben zeigten bei Anwendung des Radialimmundiffusionsverfahrens (RID) nach Mancini eine starke Abnahme der Serum-IgG1-Spiegel beim Stehen der Serumproben an der Luft. Dieser Effekt setzt praktisch sofort nach der Blutabnahme ein und beträgt nach sieben Stunden durchschnittlich 20 %, nach 36 Stunden mindestens 25 %, wobei die Bestimmungen an Blutproben gesunder Spender beiderlei Geschlechts durchgeführt werden.

Das Ausmaß des Effektes hängt unter anderem von der Höhe des Ausgangswertes ab: Proben mit hohem IgG1-Wert zeigen in der Regel ein stärkeres Absinken des Wertes, allerdings dürften nach bisherigen Vorbefunden auch noch weitere Faktoren von Einfluß sein.

Ergibt sich schon aus dem bisher Gesagten, daß ohne wirksame Stabilisierung eine exakte Bestimmung des IgG1-Spiegels im Blutserum in der klinischen Praxis nicht möglich ist, so wird die Stabilisierung ein absolutes Erfordernis, wenn die Verschiebung des Verhältnisses der IgG-Subklassen zueinander im Blut Krebskranker als Tumormarker im Sinne des erfindungsgemäßen Verfahrens ausgewertet werden soll.

Die Stabilisierung mit Radikalfängern erscheinen erfolgversprechend, die besten Ergebnisse wurden jedoch eindeutig bisher durch Zusatz von SOD erreicht. Diese kann den frischen Vollblutproben oder den Serumproben nach Abtrennung des Blutzellmaterials zugegeben werden.

Sehr günstige Stabilisierungsergebnisse werden erreicht, wenn die Stabilisierung des Serums mit SOD in einem Mengenverhältnis von 100 - 1000, vorzugsweise 300 - 400 iU SOD/ml, Serum vorgenommen wird.

Günstig ist es dabei, die SOD in Form einer Lösung mit der Konzentration 5000 - 10000 iU SOD/ml zu den Serumproben zuzugeben. Die derart stabilisierten Proben werden anschließend hinsichtlich der Verschiebung des Verhältnisses der Subklasse IgG1 zu der Summe der IgG-Subklassen im Vergleich zum Normalverhältnis geprüft.

Die Veränderung des Verhältnisses der IgG-Subklassen zueinander im Blutserum konnte überraschenderweise mit dem Auftreten maligner Tumore und sogar mit der Menge und der Ausbreitung der im Körper vorhandenen Tumormasse in Zusammenhang gebracht werden, sodaß nicht nur eine qualitative, sondern auch eine quantitative Aussage über den malignen Tumor möglich ist.

Bevorzugt wird das erfindungsgemäße Verfahren daher zur extracorporalen Überwachung des Tumorgeschehens in einem Patienten eingesetzt. Ein solches Verfahren ist vor allem während oder im Anschluß an eine Therapie für den behandelnden Arzt von großem Interesse (Monitoring). Aber auch zur Gesundenuntersuchung kann das erfindungsgemäße Verfahren erfolgreich eingesetzt werden, wobei in einfacher Weise ein erster Hinweis auf ein eventuelles Tumorgeschehen erhalten werden kann.

2

Das Verfahren stellt für den Patienten keinerlei Belastung dar und ist nach bisherigen Erfahrungen zuverlässiger als die derzeit zur Verfügung stehenden Verfahren. Von diesen gilt gegenwärtig das Tumormarker-Verfahren als das am häufigsten verwendete.

Bei der Umsetzung des erfindungsgemäßen Verfahrens in die Praxis zeigte sich, daß die Berechnung des genannten Verhältnisses günstigerweise aus den Ergebnissen an sich bekannter Analysenmethoden für IgG, insbesondere chromatographischer bzw. immunologischer Methoden, erfolgt. Die Durchführung von Immunodiffusions-Assays hat sich in diesem Zusammenhang als besonders erfolgreich erwiesen. Auch einfache immunadsorptive Säulenverfahren können angewendet werden.

Weiters wurde auch die Flüssigkeitsadsorptionschromatographie eingesetzt, wobei durch Anpassung der Arbeitsbedingungen eine außerordentliche Trennschärfe der einzelnen Komponenten erzielt werden kann. Die erhaltenen Fraktogramme können qualitativ und quantitativ ausgewertet werden.

Anstelle der chromatographischen Verfahren kann gewünschtenfalls auch die Bestimmung des $\Sigma$S-Wertes als Meßgröße herangezogen werden, jenes Wertes, der für das Sulfhydryl-Disulfid-Verhältnis im Immunglobulin G maßgebend ist. Durch die Erkenntnis, daß dieses Verhältnis mit dem Verhältnis der IgG-Subklassen untereinander im Zusammenhang steht, stellt dieser Meßwert ebenfalls ein Werkzeug zur Durchführung des erfindungsgemäßen Verfahrens dar.

Bei der Abklärung der Bedingungen der Messung des $\Sigma$S-Wertes im Zusammenhang mit dem erfindungsgemäßen Verfahren wurde die Beobachtung bestätigt, daß mit hoher Treffsicherheit zwischen malignen und benignen Geschwulst-Erkrankungen unterschieden werden kann.

Bei Mamma-Carcinomen konnte auch eine Korrelation zu den einzelnen Stadien hergestellt werden. So zeigte sich beim $T_1$Stadium eine positive Reaktion von fast 65 %, die bis zu $T_4$Stadien auf 91 % stieg. Verglichen mit üblichen Tumormarkern (z.B. CEA) ist dies eine enorme Steigerung, da diese im $T_1$Stadium eine Auffindungsrate von ca. 5 % besitzen. Es wird diesbezüglich auf die Veröffentlichung M.Smola, W.Estelberger, M.Reiter, K.Schauenstein und E.Schauenstein: CANCER, Vol.68, No.5,1991, p.1026 verwiesen.

Nach chirurgischen Eingriffen normalisierte sich der $\Sigma$S-Wert. Je nachdem, ob der chirurgische Eingriff erfolgreich war oder nicht, blieb der Wert normal oder wurde wieder pathologisch. M.Lahousen, E.Schauenstein, et al. Wiener Klin.Wschr.101,858 (1989). Es wird somit vermutet, daß der $\Sigma$S-Wert ein Maß für den humoralen Immunstatus, d.h. für die Immunabwehr darstellt.

Eine andere Möglichkeit zur Bestimmung des Verhältnisses der IgG-Subklassen zueinander besteht in der Methode der Radialimmundiffusion (RID).

Für alle Methoden gilt jedoch gleichermaßen die Tatsache, daß die Ergebnisse nur dann aussagekräftig sind, wenn vorher eine Stabilisierung der Proben vorgenommen wurde.

Als Ursache für die Instabilität des IgG1-Spiegels wird die Bildung nicht-kovalent vernetzter Aggregate angenommen, die z.B. mit RID nicht detektierbar sind. Die bisherigen Befunde sprachen dafür, daß diese Aggregatbildung durch sauerstoffhaltige Radikale ausgelöst wird und tatsächlich erwiesen sich die Stabilisierungsversuche mit SOD als besonders erfolgreich.

Zur Durchführung dieser Versuche wurde aus dem Vollblut gesunder Spender durch Stehenlassen bis zur vollständigen Gerinnung und Abzentrifugieren des Niederschlags das Serum gewonnen und dieses entweder mit oder ohne SOD sofort oder nach 36 h auf IgG1 geprüft.

Bei den ersten Laborversuchen erfolgte diese Prüfung durch. RID. Es zeigte sich, daß bei gleicher Anfangskonzentration an IgG1 die mit SOD versetzten Proben nach 36 h noch fast die gleiche Konzentration hatten, während die nicht stabilisierten Proben bis nahe an die Hälfte des Anfangswertes abgesunken waren.

Bei der Auftrennung des IgG in seine Fraktionen durch Optimierungsversuche mit Protein-A-Sepharose ergab sich, daß es durch Herabsetzen der Durchflußgeschwindigkeit im Prinzip möglich ist, zu Reinheitsgraden von 96 bis 100 % der Immunglobulin-Bestandteile zu kommen.

Basierend auf diesen Forschungsergebnissen wird gemäß der vorliegenden Erfindung auch ein Kit zur Durchführung des erfindungsgemäßen Verfahrens zur Verfügung gestellt, der durch das Stabilisierungsreagens SOD und eine Anordnung zur Bestimmung des Verhältnisses der Subklasse IgG1 zu den anderen IgG-Subklassen gekennzeichnet ist.

Die folgenden Beispiele sollen zur Erläuterung des erfindungsgemäßen Verfahrens dienen, ohne dieses in irgendeiner Weise zu beschränken.

Beispiel 1

Protokoll eines Stabilisierungsversuches:
10 ml Vollblut werden aus der Armvene eines gesunden Spenders entnommen. Nach 5 bis 10 Minuten ist die Gerinnung abgeschlossen. Es wird 10 Minuten bei 440 x g zentrifugiert. Das Serum wird abpipettiert und in 4 aliquote Anteile zu je 0,1 ml aufgeteilt:

2 Nullzeitproben "0-" und "0+" sowie 2 Proben zur Prüfung nach 36 Stunden "36-" und "36+". Die Proben "0+" und "36+" werden mit 5µl SOD-Lösung (35 iU) versetzt (SOD Lot 100H9311 von Fa. Sigma über Fa. Biotrade). Die beiden Nullproben werden mit RID-Puffer 1 : 50 verdünnt (0,05 M Tris, 0,10 M NaCl, 1 g NaN₃/l, verdünnte HCl ad pH 8,0). Die Proben "36+" und "36-" werden unverdünnt 36 Stunden bei Raumtemperatur im verschlossenen Zustand bei Tageslicht aufbewahrt. Die Proben "0-" und 0+" werden sofort auf die RID-Platte gebracht, wobei 3µl/well eingebracht werden. Agarosegel für RID-Platten:

5 ml 1-%ige Agaroselösung, 0,5 ml RID-Puffer, 0,5 ml anti-IgG1-Serum (Fa. Binding Site, PE006). Auf die gleiche Platte werden vier Verdünnungen des Referenzserums (Fa. Binding Site, BP062) aufgetragen: Verdünnungen 1:10, 1:15, 1:25 und 1:50. Nach 36 Stunden werden die Proben "36-" und "36+" nach Verdünnung mit RID-Puffer 1:50 ebenfalls auf die Platte gebracht. Sodann werden die Flächen der Präzipitatringe von "0-" und "0+" sowie der Verdünnungen des Referenzserums gemessen, zwei Tage später die der 36 Stunden-Proben. Dann werden die Werte der bei den Verdünnungen des Referenzserums gemessenen Flächen der Präzipitatringe gegen die bekannten IgG1-Konzentrationen der Verdünnungen aufgetragen. In das erhaltene Eichdiagramm werden schließlich die Flächen der aus den vier Proben erhaltenen Präzipitatringe eingetragen und daraus die IgG1-Konzentrationen durch Interpolation ermittelt.

## Tabelle 1

| Spender | Probe 0- | Probe 0+ | 36- | 36+ |
|---|---|---|---|---|
| | IgG1 (mg/ml Serum) | | | |
| G.R. | 10,24 | 10,00 | 6,59 | 9,76 |
| H.P. | 8,91 | 8,99 | 5,72 | 8,89 |
| F.T. | 7,26 | 7,26 | 3,81 | 7,03 |

Beispiel 2

Bei einem 7-stündigen Versuch ähnlicher Art wie in Beispiel 1 wurden folgende Ergebnisse erhalten:

## Tabelle 2

| | IgG1 (mg/ml Serum) | | | |
|---|---|---|---|---|
| | 0 h | 0 h+SOD | 7 h | 7 h+SOD |
| Serum 1 | 6,77 | 6,97 | 6,38 | 6,97 |
| Serum 2 | 5,45 | 5,45 | 4,55 | 5,45 |

Durch die gesamten bisherigen Versuche hat sich herausgestellt, daß unter den beschriebenen Bedingungen die Hemmwirkung von SOD mit 36 bis 40 Stunden begrenzt ist, d.h. daß die IgG-Bestimmungen auf jeden Fall innerhalb dieses Zeitraumes durchgeführt werden sollten.

Beispiel 3

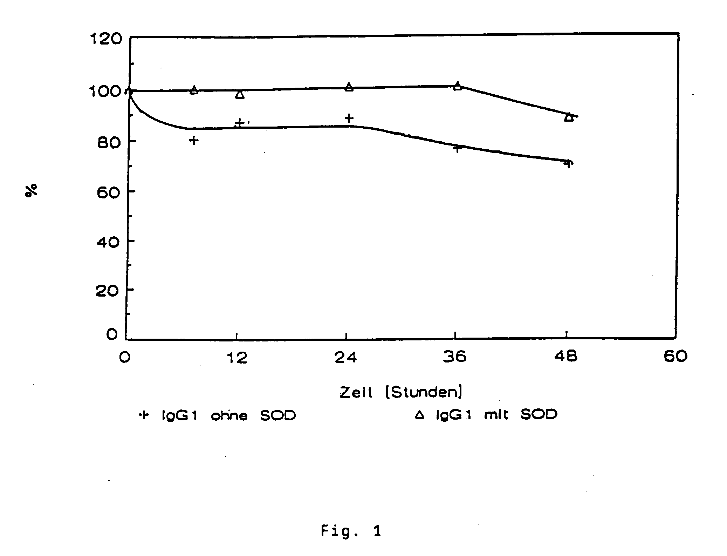

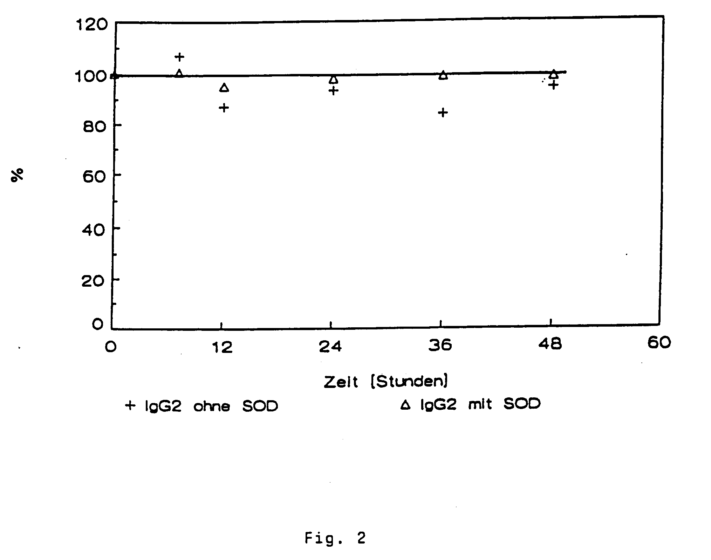

Mit jeder Serumprobe (0,1 ml) wurden Zeitreihen angesetzt, in denen der Spiegel an IgG1 und IgG2 ohne und nach Zusatz von SOD (5 µl mit 350 iU) mit RID gemessen wurde. Die Auswertung erfolgte in der Weise, daß die nach den verschiedenen Zeiten erhaltenen Meßwerte in Prozenten des Ausgangswertes ausgedrückt wurden. Dabei ergab sich für IgG1 bzw. IgG2 ein zeitlicher Verlauf, wie er aus den Fig.1 bzw. 2 der angeschlossenen Zeichnungen hervorgeht.

Diskussion der Fig.1 bis 4 der angeschlossenen Zeichnungen:

1. Die mit RID meßbare Abnahme des IgG1-Spiegels beträgt nach 48 Stunden Stehzeit (unter Verschluß, aerob, Raumtemperatur) im Mittel 30 %.

2. Der Effekt setzt offenbar prompt ein und erreicht bereits nach 7 Stunden rund 20 % Abnahme. Die Kinetik läßt hier auf eine Exponentialfunktion schließen und die Experimente haben gezeigt, daß die relative zeitliche Abnahme von IgG1 der jeweils reagierenden Menge an dieser Subklasse direkt proportional ist. Damit konnte eine der Michaelis-Menten-Beziehung entsprechende Kinetik ausgeschlossen und die Erkenntnis abgeleitet werden, daß hier eine Reaktion der zweiten Ordnung eintritt, aller Wahrscheinlichkeit nach die primäre Bildung von Dimeren, aus denen im weiteren Verlauf dann höhere Aggregate entstehen. Die Bildung solcher Aggregate würde es auch verständlich machen, daß diese in der RID nicht detektierbar sind, nachdem gezeigt werden konnte, daß Hitze-Aggregate in der RID keine erkennbaren Präzipitationskreise bilden.

3. Ob es im weiteren Verlauf der Reaktion, zwischen 12 und 24 Stunden, zu einer vorübergehenden Selbststabilisierung des Systems kommt oder ob hier eine Streuung der Meßpunkte vorliegt, kann nicht mit Sicherheit entschieden werden. Fest steht nur, daß der Prozeß anschließend kontinuierlich weiterläuft.

4. Die Hemmbarkeit der Reaktion durch SOD läßt darauf schliessen, daß es sich um eine durch O-Radikale induzierte Selbstaggregation handeln dürfte. Nach Wickens et al. (Biochim. Biophis. Acta, Vol.742:607-616, 1983) führt Bestrahlung mit kleinen UV-Dosen zur Selbstaggregation und solche Aggregate, die übrigens auch durch aktivierte Leukozyten induziert werden, zeigen eine beträchtliche Abschwächung der Präzipitatlinien.

5. Aus Fig.1 geht hervor, daß der IgG1-Spiegel im Durchschnitt innerhalb von 24 Stunden um etwa 15 %, nach 36 Stunden um 22 % abnimmt. Innerhalb dieser Zeitspanne kann die Abnahme durch SOD-Zusatz praktisch vollständig gehemmt werden. Die Bedeutung dieses Ergebnisses wird offenbar, wenn man berücksichtigt, daß in Fig.1 die mittleren prozentuellen Abnahmen dargestellt sind und daß die bei den einzelnen Proben gemessenen individuellen Abnahmen unter Umständen erheblich größer sein können, wie die folgende Tabelle 3 veranschaulicht.

## Tabelle 3

| IgG1 | IgG1+SOD | IgG1 | IgG1+SOD | IgG1 | IgG1+SOD | IgG1 | IgG1+SOO |
|------|----------|------|----------|------|----------|------|----------|
| 0 h | | 24 h | | 36 h | | 48 h | |
| (mg/ml Serum) | | | | | | | |
| 12,16 | 12,67 | 9,73 | 10,67 | ---- | ---- | 7,46 | 9,02 |
| 8,91 | 8,49 | 6,98 | 8,11 | 5,72 | 8,89 | 5,37 | 9,48 |
| 10,09 | 9,71 | 8,69 | 10,09 | 5,72 | 9,48 | 5,04 | 6,45 |
| 10,71 | 9,89 | 11,13 | 10,92 | 6,97 | 9,92 | 4,87 | 9,28 |
| 8,43 | 9,41 | 7,72 | 8,69 | 6,35 | 8,69 | 5,05 | 5,26 |
| 7,26 | 7,26 | 4,41 | 7,96 | 3,81 | 7,03 | 2,28 | 6,35 |

Beispiel 4

Im Rahmen dieses Beispiels wurden 16 Serumproben gesunder Spender untersucht. Als erstes wurde der Spiegel an IgG1 und IgG2 mit RID gemessen. Die erhaltenen Werte sind in der folgenden Tabelle 4 zusammengefaßt:

Tabelle 4

| IgG1 | IgG1 mit SOD | IgG2 | IgG2 mit SOD |
|---|---|---|---|
| | mg/ml Serum | | |
| 6,77 | 6,97 | 3,13 | 3,29 |
| 5,45 | 5,45 | 2,64 | 2,79 |
| 8,57 | 8,57 | 3,34 | 3,20 |
| 8,34 | 8,80 | 1,67 | 1,55 |
| 8,12 | 8,35 | 3,20 | 2,94 |
| 12,16 | 12,67 | 4,18 | 4,33 |
| 9,03 | 8,34 | 2,94 | 2,80 |
| 6,83 | 6,83 | 2,63 | 2,79 |
| 11,15 | 11,39 | 3,56 | 3,73 |
| 9,95 | 9,48 | 4,38 | 5,42 |
| 13,41 | 12,90 | 2,33 | 2,04 |
| 8,91 | 8,49 | 3,60 | 3,59 |
| 10,09 | 9,71 | 3,77 | 3,60 |
| 10,71 | 9,89 | 6,56 | 6,97 |
| 8,43 | 9,41 | 3,55 | 3,85 |
| 7,26 | 7,26 | 1,48 | 1,72 |
| AVG: 9,07 | 9,03 | 3,31 | 3,41 |
| SEM: 0,52 | 0,51 | 0,29 | 0,34 |

Zusätzlich konnte noch auf 6 weitere Werte zurückgegriffen werden, die in Tabelle 5 zusammengestellt sind.

Tabelle 5

| IgG1 | IgG2 |
|---|---|
| mg/ml Serum | |
| 6,79 | 3,04 |
| 7,09 | 2,45 |
| 5,86 | 1,43 |
| 6,82 | 1,61 |
| 5,76 | 2,79 |
| 5,75 | 2,65 |
| AVG: 6,35 | 2,33 |
| SEM: 0,25 | 0,27 |

Mittelt man die erhaltenen Mittelwerte, so resultieren für die 22 Gesund-Seren 8,33 ± 0,48 mg IgG1 und 3,04 ± 0,25 mg IgG2/ml Serum.

Die IgG1-Spiegel von Krebsseren liegen im Mittel um 23 bis 24 % tiefer als in Normalseren, sodaß die absolute Notwendigkeit einer effizienten Hemmung evident wird. SOD erfüllt diese Bedingung, allerdings nur bis zu einer Stehzeit der Probe von rund 40 Stunden, was aber für die meisten Fälle wohl ausreichend sein dürfte. Bemerkenswerterweise tritt ein eindeutig definierter Zeiteffekt, wie er in Fig.1 dargestellt ist, nur bei IgG1 auf, während IgG2, die zweite Hauptkomponente, unregelmäßige Schwankungen des Spiegels zeigt, die jedoch durch SOD ebenfalls unterdrückt werden (Fig.2). Da nun SOD das in der RID angezeigte Absinken von IgG1 hemmt und nur diese Subklasse eine reaktive Disulfidbrücke SS∗ im Molekül enthält, wird verständlich, daß SOD auch für die Messung des ΣS-Wertes, der ja zu 85 % aus dem Term für SS∗ besteht, als Stabilisator eingesetzt werden kann.

## Beispiel 5

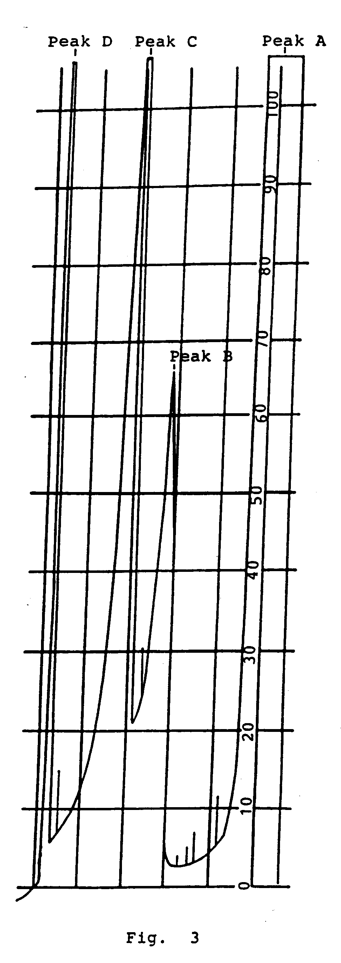

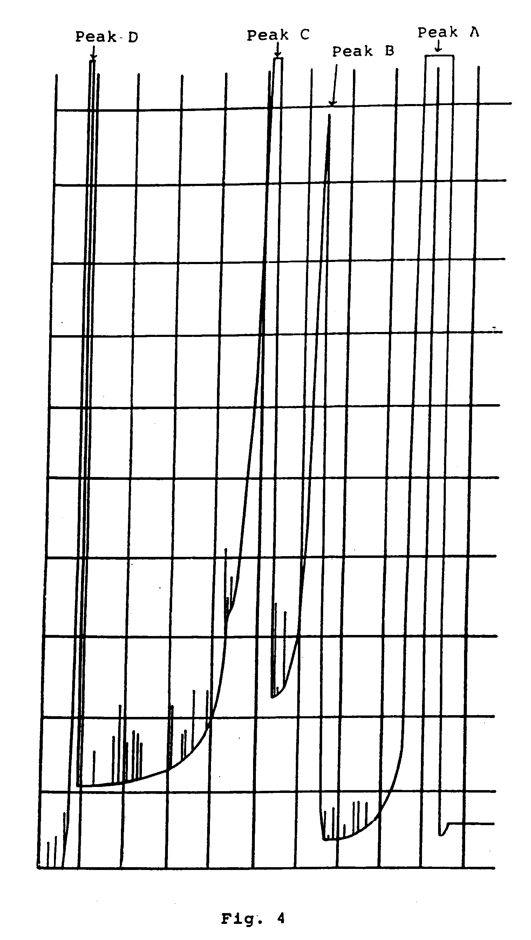

Trennung der IgG-Subklassen 1 und 2 an Protein-A-Sepharose-Säulen.
Pufferzusammensetzung:
0,05 M Phosphat-Citrat-Puffer pH 4,7
0,086 M Phosphat-Citrat-Puffer pH 4,3
Durchflußgeschwindigkeiten variierend von 70 ml/h bis 10 ml/h.
Es wurden die angeschlossenen Fraktogramme (Fig. 3 und 4) erhalten, bei denen in den relevanten Peaks B und C Reinheitsgrade der Komponenten bis zu 96 bzw. 100 % erzielt wurden.

## Beispiel 6

### Bestimmung der IgG1- und IgG2-Konzentration einerseits durch Affinitätschromatographie mit Protein-A-Sepharose, andererseits, zur Kontrolle, mit RID

Für den Vergleich der beiden Methoden wurden Seren von 20 gesunden Probanden sowie Seren von 40 Patientinnen mit Ovarial-, Cervix- und Collum-Karzinomen verwendet, wofür die Blutproben präoperativ abgenommen wurden.

Die Blutproben (2-10 ml) wurden nach Abnahme sofort bei 440 x g 10 Minuten zentrifugiert, das Serum abpipettiert, 1:50 mit RID-Puffer verdünnt und auf die RID-Platten aufgetragen; nach 2 Tagen wurden die Präzipitatringe vermessen und die IgG1- und IgG2-Konzentration mittels Referenzserum bestimmt (Mancini-Technik).

Für die Isolierung von IgG1 und IgG2 mittels Protein-A Sepharose wurde 0,3 ml Serum auf die Säule (K 9/15) aufgetragen und mit 0.16 M Phosphat-Citrat-Puffer pH 7.0 und einer Durchlaufgeschwindigkeit von 10 ml/h eluiert. Hierbei bindet IgG über den Fc-Teil an das Protein-A und die restlichen Serumproteine werden ungebunden eluiert. Außer IgG wird auch IgM und IgA an Protein-A gebunden. Nach Erreichen der Baseline kommt es zum Pufferwechsel: zunächst wird IgG2 mit 0.05 M Phosphat-Citrat-Puffer pH 4.7 und danach IgG1 mit 0.086 M Phosphat-Citrat-Puffer pH 4.3 eluiert. Danach erfolgt die Reinigung des Gels mittels 0.1 M Citrat-Puffer und dann erfolgt der Austausch des Citrat-Puffers gegen den Startpuffer (0.16 M Phosphat-Citrat-Puffer pH 7.0). Die Eluate von IgG1 und IgG2 werden mit 1 N NaOH auf PH 7.0 titriert und die Proteinkonzentrationen photometrisch bei 278 nm bestimmt. Der Extinktionskoeffizient von $\varepsilon' = 1.58$ ermöglicht dann die Konzentrationsbestimmung.

Tabelle 6 listet die Mittelwerte der IgG1- und IgG2-Konzentrationen der Serumproben ($\pm$ sem), sowohl mit RID als auch mit Protein-A bestimmt, auf. Aus diesen beiden Werten kann dann der Quotient G1/G2 gebildet werden.

### Tabelle 6a

**bestimmt mit RID**

|  | IgG1 | IgG2 | G1/G2 | n |
|---|---|---|---|---|
|  | (mg/ml Serum) | | | |
| Ges. Probanden | $8.72 \pm 0.41$ | $3.66 \pm 0.31$ | $2.62 \pm 0.21$ | 20 |
| Gyn. Tumore | $6.70 \pm 0.50$ | $3.69 \pm 0.35$ | $2.04 \pm 0.14$ | 40 |

7

## Tabelle 6b

### bestimmt mit Protein-A

| IgG1 | IgG2 | G1/G2 | |
|---|---|---|---|
| | (mg/ml Serum) | | n |
| $7.27^{\pm}0.36$ | $3.14^{\pm}0.25$ | $2.54^{\pm}0.22$ | 20 |
| $5.75^{\pm}0.37$ | $3.16^{\pm}0.29$ | $2.08^{\pm}0.13$ | 40 |

Mit RID bestimmt, unterscheiden sich die Mittelwerte der IgG1-Konzentrationen der gesunden Probanden und Tumorpatienten hochsignifikant ($p = 0.005$), wobei 27.5 % falsch negative und 25 % falsch positive Resultate erhalten wurden. Ein ähnliches Ergebnis zeigt die Isolierung von IgG1 mit Protein-A: $p = 0.005$ und 30 % falsch negative und 35 % falsch positive Resultate.

**Patentansprüche**

1. Verfahren zur Feststellung maligner Tumore bzw. zur Bestimmung ihrer Tumormasse anhand von Eigenschaften des Serum-Immunglobulins G (IgG) des Patienten, dadurch gekennzeichnet, daß die frischen Blut- oder Serumproben des Patienten mit Radikalfängern, vorzugsweise mit Superoxiddismutase (SOD), stabilisiert werden und im Serum der Proben die Verschiebung des Verhältnisses mindestens einer der IgG-Subklassen zu der Summe der IgG-Subklassen im Vergleich zum Normalverhältnis bestimmt wird.

2. Verfahren nach Anspruch 1, dadurch gekennzeichnet, daß im Serum der stabilisierten Proben die Verschiebung des Verhältnisses der Subklasse IgG1 und bzw. oder der Subklasse IgG2 zu der Summe der IgG-Subklassen im Vergleich zum Normalverhältnis bestimmt wird.

3. Verfahren nach Anspruch 1 oder 2, dadurch gekennzeichnet, daß die genannte Verschiebung des Verhältnisses durch Bestimmung des $\Sigma$S-Wertes bestimmt wird.

4. Verfahren nach Anspruch 1 oder 2, dadurch gekennzeichnet, daß die genannte Verschiebung des Verhältnisses aus den Ergebnissen an sich bekannter Analysenmethoden für IgG, insbesondere chromatographischen bzw. immunologischen Methoden, berechnet wird.

5. Verfahren nach Anspruch 4, dadurch gekennzeichnet, daß die genannte Verschiebung des Verhältnisses durch einen Immundiffusions-Assay bestimmt wird.

6. Verfahren nach einem der Ansprüche 1 bis 5, dadurch gekennzeichnet, daß die Stabilisierung des Serums mit SOD in einem Mengenverhältnis von 100 bis 1000, vorzugsweise 300 bis 400, iU SOD/ml Serum vorgenommen wird.

7. Verwendung des Verfahrens nach einem der Ansprüche 1 bis 6 zur extracorporalen Erfassung eines Tumorgeschehens in einem Patienten.

8. Kit zur Durchführung des Verfahrens nach einem der Ansprüche 1 bis 7, gekennzeichnet durch ein Stabilisierungsreagens, vorzugsweise SOD, und eine Anordnung zur Bestimmung des Verhältnisses mindestens einer IgG-Subklasse zu der Summe der IgG-Subklassen.

**Claims**

1. A method for diagnosing malignant tumours or determining their mass with the aid of properties of the patient's serum immunoglobulin G (IgG), characterized in that the patient's fresh blood or serum samples are stabilised with radical traps, preferably with superoxide dismutase (SOD) and in the sample serum the shift in the ratio of at least one of the IgG subclasses to the sum of the IgG subclasses is determined in comparison with the normal ratio.

2. A method according to claim 1, characterized in that in the serum of the stabilised samples the shift in the ratio of the subclass IgG1 and/or the subclass IgG2 to the sum of the IgG subclasses is determined in comparison with the normal ratio.

3. A method according to claim 1 or claim 2, characterized in that the above-mentioned shift in ratio is determined by determination of the ōS value.

4. A method according to claim 1 or claim 2, characterized in that the above-mentioned shift in the ratio is calculated from the results of analysis methods for IgG known per se, especially chromatographic or immunological methods.

5. A method according to claim 4, characterized in that the above-mentioned shift in the ratio is determined by an immunodiffusion assay.

6. A method according to any one of claims 1 to 5, characterized in that stabilisation of the serum is effected with SOD in a quantity ratio of from 100 to 1000, preferably 300 to 400, IU SOD/ml serum.

7. Use of the method according to any one of claims 1 to 6 for extracorporeal detection of the occurrence of a tumour in a patient.

8. A kit for carrying out the method according to any one of claims 1 to 7, characterized by a stabilising reagent, preferably SOD, and an arrangement for determining the relaionship of at least one IgG subclass to the sum of the IgG subclasses.

**Revendications**

1. Procédé pour détecter des tumeurs malignes ou déterminer leur masse tumorale à l'aide des propriétés de l'immunoglobuline G (IgG) du sérum du patient, caractérisé en ce qu'on stabilise les échantillons frais de sang ou de sérum du patient à l'aide de capteurs de radicaux, de préférence avec de la superoxyde-dismutase (SOD), et que l'on détermine, dans le sérum des échantillons, le déplacement, par rapport à la valeur normale du rapport, du rapport entre au moins l'une des sous-classes d'IgG et la somme des sous-classes d'IgG.

2. Procédé selon la revendication 1, caractérisé en ce que, dans le sérum des échantillons stabilisés, on détermine, par rapport à la valeur normale du rapport, le déplacement du rapport entre la sous-classe IgG1 et/ou de la sous-classe IgG2 et la somme des sous-classes d'IgG.

3. Procédé selon la revendication 1 ou 2, caractérisé en ce qu'on détermine ledit déplacement du rapport par une détermination de la valeur $\Sigma S$.

4. Procédé selon la revendication 1 ou 2, caractérisé en ce que ledit déplacement du rapport est calculé à partir des résultats de méthodes connues en soi d'analyse des IgG, en particulier les méthodes chromatographiques ou immunologiques.

5. Procédé selon la revendication 4, caractérisé en ce qu'on détermine ledit déplacement du rapport par une analyse par immunodiffusion.

6. Procédé selon l'une des revendications 1 à 5, caractérisé en ce qu'on procède à la stabilisation du sérum avec du SOD selon un rapport pondéral de 100 à 1000 et de préférence de 300 à 400 UI SOD/ml.

7. Utilisation du procédé selon l'une des revendications 1 à 6, pour la détection extracorporelle d'un phénomène tumoral chez un patient.

8. Trousse pour la mise en oeuvre du procédé selon l'une des revendications 1 à 7, caractérisée par un réactif de stabilisation, de préférence le SOD, et un arrangement destiné à déterminer le rapport entre au moins une sous-classe d'IgG et la somme des sous-classes d'IgG.

Fig. 1

Fig. 2

Fig. 3

Fig. 4